## Europäisches Patentamt
## European Patent Office
## Office européen des brevets

(11) Veröffentlichungsnummer: **0 198 415**
**B1**

(12) **EUROPÄISCHE PATENTSCHRIFT**

(45) Veröffentlichungstag der Patentschrift:
**14.06.89**

(21) Anmeldenummer: **86104879.1**

(22) Anmeldetag: **09.04.86**

(51) Int. Cl.⁴: **C 12 N 15/00, C 12 N 1/20, C 12 P 21/00 // (C12N1/20, C12R1:19)**

(54) Veränderung der DNA-Sequenz zwischen Shine-Dalgarno-Sequenz und Startcodon des trp-Operons zur Steigerung der Proteinexpression.

(30) Priorität: 19.04.85 DE 3514113

(43) Veröffentlichungstag der Anmeldung:
22.10.86 Patentblatt 86/43

(45) Bekanntmachung des Hinweises auf die Patenterteilung:
14.06.89 Patentblatt 89/24

(84) Benannte Vertragsstaaten:
AT BE CH DE FR GB IT LI LU NL SE

(73) Patentinhaber: HOECHST AKTIENGESELLSCHAFT,
Postfach 80 03 20, D-6230 Frankfurt am Main 80 (DE)

(72) Erfinder: Engels, Joachim, Prof. Dr., Feldbergstrasse 1,
D-6242 Kronberg/Taunus (DE)
Erfinder: Leineweber, Michael, Dr., Helmchenweg 74,
D-6230 Frankfurt am Main 80 (DE)
Erfinder: Uhlmann, Eugen, Dr., Zum Talblick 31,
D-6246 Glashütten/Taunus (DE)
Erfinder: Wengenmayer, Friedrich, Dr., Am
Seyenbach 38, D-6238 Hofheim am Taunus (DE)

(56) Entgegenhaltungen:
NATURE, Band 291, 11. Juni 1981, Seiten 503-506,
Macmillan Journals Ltd., London, GB; J.C. EDMAN et
al.: "Synthesis of hepatitis B surface and core antigens
in E. coli"
NUCLEIC ACIDS RESEARCH, Band 11, Nr. 17,
September 1983, Seiten 5837-5854, IRL Press Ltd,
Oxford, GB; N. WARBURTON et al.: "Increased
expression of a cloned gene by local mutagenesis of its
promoter and ribosome binding site"
CHEMICAL ABSTRACTS, Band 96, Nr. 21, 24. Mai 1982,
Seite 174, Zusammenfassung Nr. 175316s, Columbus,
Ohio, US; H.M. SHEPARD et al.: "Increased synthesis in
E. coli of fibroblast and leukocyte interferons through
alterations in ribosome binding sites"
CHEMICAL ABSTRACTS, Band 101, Nr. 1, 2. Juli 1984,
Seite 163, Zusammenfassung Nr. 1814j, Columbus,

(56) Entgegenhaltungen: (Fortsetzung)
Ohio, US; S. ITOH et al.: "Efficient expression in
Escherichia coli of a mature and a modified human
interferon-beta1"
GENE, Band 36, Nr. 3, 1985, Seiten 375-379, Elsevier
Science Publishers, Amsterdam, NL; E.A. WHITEHORN
et al.: "The effects of hybrid ribosome-binding-site
variants on the expression of human interferon-beta in
Escherichia coli"

Die Akte enthält technische Angaben, die nach dem
Eingang der Anmeldung eingereicht wurden und die
nicht in dieser Patentschrift enthalten sind.

**Beschreibung**

Regulationssequenzen des trp-Operons werden vielfach zur Expression eukaryotischer Proteine in E. coli verwendet. Ein den Promotor und den Operator des trp-Operons enthaltender DNA-Abschnitt ist inzwischen handelsüblich.

Modifikationen der Regulationssequenzen des trp-Operons sind schon bekannt. So ist beispielsweise in der Deutschen Offenlegungsschrift 3 247 922 beschrieben, wie in die Nucleotid-Sequenz der Regulationselemente des trp-Operons aus Serratia marcescens zwischen ribosomaler Bindungsstelle und Startcodon ein Hind III-Schnittstelle eingebaut werden kann. Aus J.C. Egdman et al., Nature 291 (1981) 503–506, ist es bekannt, in die entsprechende Sequenz aus E. coli eine Cla I-Schnittstelle einzufügen. Diese Variation verändert jedoch die Zahl der Nucleotide zwischen ribosomaler Bindungsstelle und Startcodon gegenüber der natürlichen Sequenz.

Es wurde nun gefunden, daß durch den Austausch eines einzigen Nucleotids, also ohne Veränderung der Nucleotidanzahl, eine Schnittstelle für ein Restriktionsenzym in die DNA-Sequenz zwischen ribosomaler Bindungsstelle und Startcodon eingefügt werden kann. Erfindungsgemäß wird das unmittelbar vor dem Startcodon angeordnete Nucleosid Adenosin durch Cytidin ersetzt.

Die Erfindung betrifft somit einen modifizierten DNA-Abschnitt des trp-Operons von E. coli, der zwischen der «Shine-Dalgarno»-Sequenz und dem ersten Startcodon liegt und gekennzeichnet ist durch die DNA-Sequenz I

5′   GTATCGACC   3′       (I)
3′   CATAGCTGG   5′

Diese Sequenz I schließt am 5′-Ende des oberen Stranges an die «Shine-Dalgarno»-Sequenz

5′   AAGG   3′
3′   TTCC   5′

des trp-Operons an (die der eigentlichen ribosomalen Bindungsstelle auf der Ebene der RNA entspricht), und am 3′-Ende des oberen Stranges an das Startcodon ATG.

Im folgenden wird die erfindungsgemäße Sequenz I der natürlichen E. coli-Sequenz und der aus Edman et al., a.a.O., bekannten Sequenz gegenübergestellt, wobei – aus Gründen der Übersichtlichkeit – nur der obere Strang, im folgenden «codierender Strang» genannt, wiedergegeben ist:

| E. coli | GTA TCG ACA |
| Edman et al. | GTA TCG AT |
| Sequenz I | GTA TCG ACC |

(Änderungen gegenüber der E. coli-Sequenz sind unterstrichen).

Dieser erfindungsgemäße Austausch von C gegen das A der natürlichen Sequenz bringt folgende Vorteile mit sich:

Folgt im Anschluß an das Startcodon ATG das Nucleosid Guanosin, so wird dadurch die Erkennungssequenz C↓CATGG für das Restriktionsenzym Nco I gebildet.

Diese Schnittstelle erlaubt die Insertion von DNA unmittelbar in der Nachbarschaft zum Startcodon, beispielsweise mit Hilfe synthetischer Oligonucleotide der Formel II

5′   CATGX...   3′       (II)
3′   Y...       5′

in der X und Y das erste komplementäre Nucleotidpaar nach dem Startcodon eines Strukturgens bedeuten. Wenn hierbei X für G und Y für C stehen, ist in dem Ligierungsprodukt die Nco I-Schnittstelle erhalten geblieben. Wird dagegen beispielsweise ein synthetischer Linker eingesetzt, dessen überhängende Sequenz 5′ CATG 3′ an ein anderes Nucleotid anschließt, so ist zwar die Nco I-Schnittstelle aufgehoben, dafür aber die volle Variabilität hinsichtlich der ersten Aminosäure nach dem Startcodon gegeben.

Man kann natürlich auch die überstehenden Enden der mit NcoI geschnittenen DNA enzymatisch auffüllen, z.B. mittels Klenow-Polymerase, und die so erhaltene stumpfendige DNA der Sequenz III

5′   GTA TCG ACC ATG   3′
3′   CAT AGC TGG TAC   5′       (III)

mit einer stumpfendigen Sequenz IV

5′   X...   3′       (IV)
3′   Y...   5′

zur DNA-Sequenz V

5′   GTA TCG ACC ATG X ...   3′
3′   CAT AGC TGG TAC Y ...   5′       (V)

ligieren, wobei X und Y die genannten Bedeutungen haben. Hierbei wird kein weiteres Startcodon für ein bestimmtes zu exprimierendes Strukturgen benötigt. Dies ist beispielsweise zur Herstellung von am N-terminalen Ende verkürzten Proteinen günstig.

Möglich ist auch ein enzymatischer Abbau der überstehenden Enden, wobei ebenfalls eine stumpfendige DNA-Sequenz VI

5′   GTA TCG AC   3′(VI)
3′   CAT AGC TG   5′

entsteht, die mit einer stumpfendigen Sequenz VII

5′   Z ATG X ...   3′
3′   Z′ TAC Y ...   5′       (VII)

zur DNA-Sequenz VIII

5′   GTA TCG ACZ ATG X ...   3′
3′   CAT AGC TGZ′ TAC Y ...   5′       (VIII)

ligiert werden kann, wobei Z und Z' ein beliebiges Nucleotidpaar bedeuten, das auch entfallen kann. Wenn Z und Z' für A bzw. T stehen, wird die natürliche E. coli-Sequenz gebildet.

Außer diesen vielfältigen Klonierungsmöglichkeiten bietet die Erfindung den Vorteil, daß die Expression des Strukturgens in überraschendem Ausmaß verbessert wird.

Ein anderer Aspekt der Erfindung betrifft ein Verfahren zur Herstellung eines Vektors mit dem trp-Expressionssystem aus E. coli, das dadurch gekennzeichnet ist, daß man einen E. coli-Vektor, der die DNA-Sequenz I (codierender Strang), gefolgt von 5' ATGG 3', enthält, mit dem Restriktionsenzym Nco I schneidet und

a) in die Schnittstelle eine Genstruktur der DNA-Sequenz II ligiert oder

b) die Schnittstelle enzymatisch auffüllt und die DNA der Sequenz III mit einer DNA der Sequenz IV ligiert oder

c) die überstehende Sequenz der Schnittstelle enzymatisch abbaut und die DNA der Sequenz VI mit einer DNA der Sequenz VII ligiert.

Ein weiterer Aspekt der Erfindung betrifft E. coli-Wirtsorganismen, die durch einen Gehalt an einem erfindungsgemäß erhaltenen Vektor gekennzeichnet sind. Weiterhin betrifft die Erfindung ein Verfahren zur Herstellung von Polypeptiden aus genetisch codierbaren Aminosäuren, das dadurch gekennzeichnet ist, daß man mit einem erfindungsgemäßen Vektor transformierte E. coli-Zellen in bekannter Weise zur Expression bringt.

Weitere Aspekte der Erfindung und ihre bevorzugten Ausführungsformen werden im folgenden näher erläutert und in den Patentansprüchen niedergelegt.

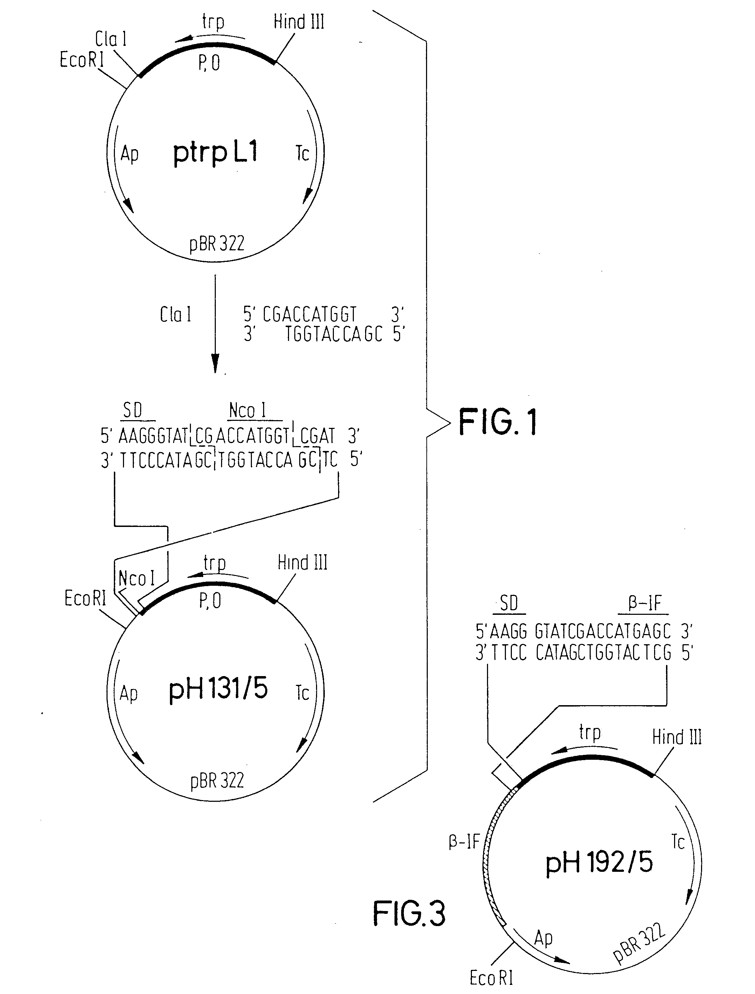

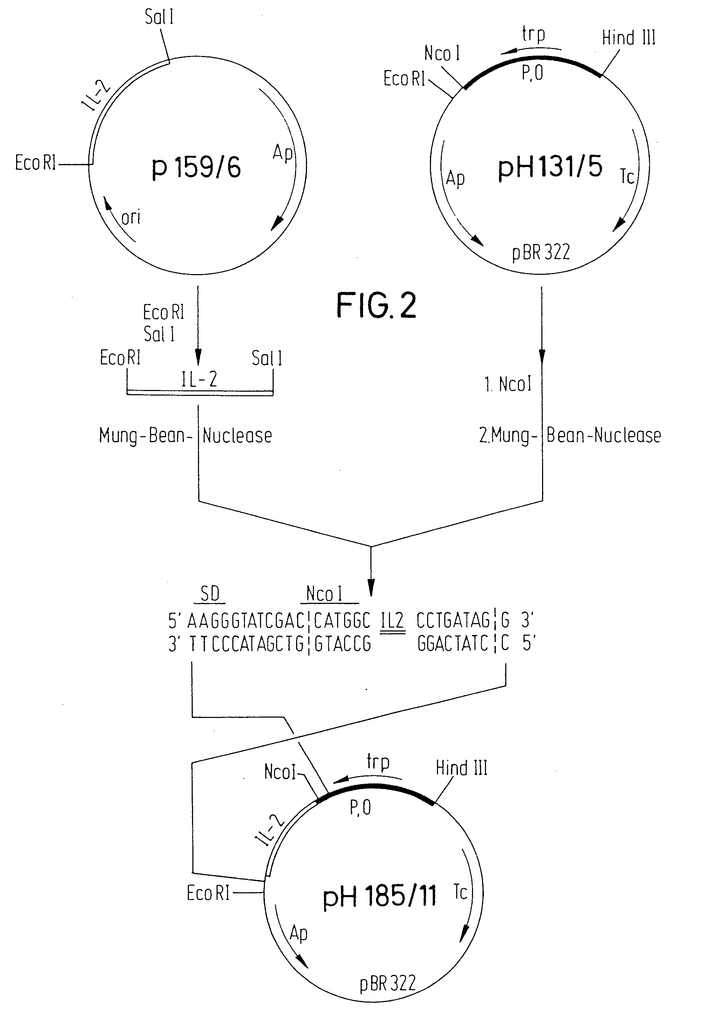

Die Figuren 1 bis 3 erläutern die Ausführungsbeispiele näher. So zeigt Figur 1 die Herstellung des Plasmids pH 131/5 aus dem bekannten Plasmid ptrpL 1. Figur 2 zeigt die Herstellung des für Interleukin-2 codierenden Plasmids pH 185/11 aus dem bekannten Plasmid p 159/6 und dem Plasmid pH 131/5 (Figur 1). Figur 3 zeigt schließlich das für β-Interferon codierende Plasmid pH 192/5.

Eine besondere Ausgestaltung der Erfindung besteht darin, daß der Vektor eine Ampicillin-Resistenz mit dem β-Lactamase-Promotor in der gleichen Orientierung wie der trp-Promotor aufweist. Es hat sich nämlich überraschenderweise gezeigt, daß durch das erfindungsgemäß modifizierte trp-Operon nicht nur eine sehr starke Expression des nach dem Startcodon angeordneten Gens erfolgt, sondern auch die β-Lactamase induziert werden kann. Eine erhöhte Konzentration der β-Lactamase verleiht eine Resistenz gegen höhere Konzentrationen an Ampicillin, wodurch eine weitere Selektionsmöglichkeit eröffnet wird. Hierdurch wird es ermöglicht, besonders günstige Promotormutationen oder auch Variationen der Nucleotide im Bereich der ribosomalen Bindungsstelle bei jedem zu exprimierenden Protein schnell zu prüfen.

Wenn die gleichzeitige Bildung von β-Lactamase unerwünscht ist, aber von Vektoren mit der Ampicillin-Resistens Gebrauch gemacht werden soll, kann diese durch Insertion eines Terminators zwischen dem Strukturgen für das erwünschte Polypeptid und dem Strukturgen für die β-Lactamase unterdrückt werden. Eine weitere Ausgestaltung dieses Aspekts der Erfindung besteht dementsprechend in der Einfügung eines geeigneten Terminators, vorzugsweise eines bakteriellen Terminators, zwischen die genannten Gene. Besonders geeignet ist der Terminator des trp-Operons, der an geeigneter Stelle eingebaut wird, beispielsweise 10 bis 20 Nucleotide nach dem Stopcodon (oder den Stopcodons) für das Strukturgen oder unmittelbar vor dem β-Lactamase-Operon.

Die erfindungsgemäße Genkonstruktion mit dem trp-Promotor/Operator kann in alle in E. coli replizierenden Plasmide eingebaut werden. Vorteilhaft verwendet man die handelsüblichen E. coli-Vektoren wie pBR 322, pBR 325, pACYC 177, pACYC 184, pUC 8 und deren Derivate. Als Derivate kommen beispielsweise solche Plasmide in Betracht, aus denen nicht essentielle Regionen entfernt, Schnittstellen oder Marker eingebaut oder verändert wurden.

Die Gensequenzen II, IV, V, VII und VIII enthalten mit dem Nucleotidpaar XY den Anfang eines beliebigen Strukturgens, beispielsweise zunächst eines wirtseigenen Gens, welches für ein Protein codiert, das den Transport in den periplasmatischen Raum oder an eine Zellmembran bewirkt. Man kann auf diese Weise Fusionsproteine herstellen, die aus dem Plasma enfernt und so leichter isoliert und/oder vom Abbau durch zelleigene Enzyme geschützt werden können. Man kann aber auch Fusionsproteine erzeugen, die auf Grund ihrer Unlöslichkeit leicht von den zelleigenen Proteinen abgetrennt werden können. Außerdem ist es möglich, die gewünschten Proteine direkt zu exprimieren, indem man das Strukturgen unmittelbar hinter das Startcodon ATG setzt.

Beispiele für Polypeptide, die erfindungsgemäß gewonnen werden können, sind Insulin, Interferone, Interleukine wie Interleukin-2, Hirudin oder Somatostation. Die Erfindung wird in den folgenden Beispielen näher erläutert. Bezüglich der einzelnen Verfahrensschritte wird auf das Lehrbuch «Molecular Cloning» von Maniatis et al., Cold Spring Harbor (1982), verwiesen.

Beispiel 1 Chromosomale E. coli-DNA wird mit Hinf I geschnitten und das 492 bp-Fragment isoliert, das aus dem trp-Operon den Promotor, Operator, das Strukturgen des L-Peptids, den Attenuator und die Kodons für die ersten sechs Aminosäuren des trp-E-Strukturgens enthält. Dieses Fragment wird mit Hilfe von Klenow-Polymerase mit Desoxynucleotidtriphosphaten aufgefüllt, an beiden Enden mit einem Oligonucleotid verbunden, das eine Erkennungssequenz für Hind III enthält, und mit Hind III nachgeschnitten. Das so erhaltene Hind III-Fragment wird in die Hind III-Schnittstelle von pBR 322 ligiert. Man erhält so das Plasmid ptrpE2-1 (J.C. Edmann et al., a.a.O.). Dieses wird wie beschrieben in das Plasmid ptrpL1 überführt.

Zur Umwandlung dieses Ausgangsprodukts in einen erfindungsgemäßen Vektor wird dieses mit Cla I nach den Empfehlungen des Herstellers

(New England Biolabs) umgesetzt. Nach beendeter Inkubation wird das Inkubationsgemisch mit Phenol extrahiert, die organische Phase abgetrennt und die DNA durch Zusatz des 2,5fachen Volumens Ethanol und Inkubation bei −20 °C ausgefällt.

Nach Abzentrifugieren der DNA wird diese mit Alkalischer Phosphatase (Boehringer Mannhein) behandelt, um 5'-Phosphat-Reste zu entfernen.

Das synthetisch hergestellte Oligonucleotid IX

5'  CGACCATGGT  3'      (IX)

wird mit dem Enzym Polynucleotid-Kinase und ATP am 5'-Ende phosphoryliert. Hierzu wird das synthetische Oligonucleotid 5 Minuten auf 70 °C erwärmt und dann sofort im Eisbad abgekühlt. Die Phosphorylierung erfolgt in 25 μl Puffer (50 mM Tris-HCl, pH 7,6; 10 mM MgCl$_2$, 5 mM Dithiothreitol (DTT) unter Zusatz von 100 μM ATP und etwa 10 Einheiten T4-Polynucleotid-Kinase bei 37 °C im Laufe von 30 Minuten. Die Reaktion wird durch Zusatz des Natriumsalzes von Ethylendiamintraessigsäure (EDTA) bis zu einer Endkonzentration von 50 μM beendet. Überschüssiges ATP kann abgetrennt werden, beispielsweise durch Gelfiltration an Sepharose ($^{(R)}$SEPHADEX G 50, fine).

Das Oligonucleotid IX ist selbst-komplementär und kann sich zur doppelsträngigen Struktur X

5'  CGACCATGGT  3'
      TGGTACCAGC      (X)

assoziieren. Dieses doppelsträngige Oligonucleotid X weist überstehende Enden auf, die eine Insertion in die Cla I-Stelle des geöffneten Plasmids ptrpL1 erlauben.

Etwa 50 ng des Oligonucleotids werden mit ca. 1 μg des umgesetzten, mit Phosphatase behandelten Plasmids in 30 μl Puffer (50 mM Tris-HCl, pH 7,4; 10 mM MgCl$_2$, 10 mM DTT) unter Zusatz von 1 mM ATP und 0,1 ng/ml Rinderserum-Albumin (RAS) bei 12 °C 20 Stunden inkubiert. Man erhält das Plasmid pH 131/5 (Figur 1).

Das Reaktionsgemisch kann unmittelbar zur Transformation kompetenter E. coli-Zellen eingesetzt werden. Die Selektion erfolgt auf Agar-Platten mit L-Broth (H.J. Miller, Experiments in Molecular Genetics, Cold Spring Harbor (1972) und 50 μg/ml Ampicillin.

Da in das Plasmid pH 131/5 eine Nco I-Schnittstelle eingeführt wurde, wurden die Ampicillin-resistenten Kolonien daraufhin überprüft, ob die enthaltene Plasmid-DNA ein etwa 300 bp großes Hind III-Nco I-Fragment enthält. Über 80% der Kolonien wies dieses Fragment auf. Die Sequenzanalyse nach Maxam-Gilbert bestätigte den Einbau des synthetischen DNA-Fragments und die angegebene Sequenz des Plasmids pH 131/5 gemäß Figur 1.

Beispiel 2
Das Plasmid p 159/6 gemäß Figur 5 der Deutschen Offenlegungsschrift 3 419 995 wird mit den Enzymen EcoR I und Sal I nach den Empfehlungen der Hersteller inkubiert und gelelektrophoretisch ein 420 bp DNA-Fragment abgetrennt, das die genetische Information für Human-Interleukin-2 enthält. Die einzelsträngigen überstehenden Enden werden mit Mung-Bean-Nuclease (Pharmacia P-L Biochemicals) unter den vom Hersteller empfohlenen Bedingungen abgebaut.

Das Plasmid pH 131/5 wird mit Nco I umgesetzt und die überstehenden einzelsträngigen Enden werden ebenfalls mit Mung-Bean-Nuclease abgebaut. Anschließend erfolgt der Einbau des nun stumpfendigen Strukturgens für Interleukin-2 in das stumpfendig gemachte geöffnete Plasmid unter «blunt end»-Bedingungen mit DNA-Ligase. Hierbei wird die Nco I-Schnittstelle rückgebildet. Nach Transformation in E. coli 294 erfolgt die Selektion der Ampicillin-resistenten Klone, die passende Restriktionsfragmente aufweisen, beispielsweise ein etwa 260 bp umfassendes Eco RI-Xba I-Fragment oder ein Eco RI-Sac I-Fragment mit etwa 150 bp.

Bei dem Plasmid pH 185/11 (Figur 2) wurde die Nucleotidsequenz durch Sequenzanalyse bestätigt. Bei dieser Konstruktion bleibt die Nco I-Restriktionsstelle erhalten.

Zur Expression des Interleukins-2 werden E. coli 294-Bakterien, die das Plasmid pH 185/11 enthalten, in LB-Medium, (H.J. Miller, a.a.O.) mit 50 μg/ml Ampicillin über Nacht unter Belüftung inkubiert. Anschließend wird eine 1:100 Verdünnung mit M 9-Medium (H.J. Millre, a.a.O.) mit 1 μg/ml Thiamin und 500 μg/ml Casaminosäuren (casamino acids) hergestellt. Bei OD = 0,5 kann mit Indolyl-3-acrylsäure bis zu einer Endkonzentration von 15 μg/ml induziert werden. Nach weiteren 2 bis 3 Stunden werden die Bakterien abzentrifugiert. Durch SDS-Gelelektrophorese kann bei den induzierten Bakterien eine starke Proteinbande festgestellt werden, die mit Antikörpern gegen ein nach der deutschen Offenlegungsschrift 3 419 995 hergestelltes Interleukin-2 reagiert. Die Bande entspricht dem erwarteten Molekulargewicht von Interleukin-2 und tritt bei nicht-induzierten Bakterien nicht auf. Die biologische Aktivität des Interleukin-2 kann in hoher Konzentration in den induzierten Bakterien nachgewiesen werden.

Die vorstehend genannten Bedingungen für die Anzucht der Bakterien gelten für Schüttelkolben. Bei der Fermentation zu höheren OD-Werten (über 3) sind höhere Konzentrationen an Casaminosäuren und/oder L-Tryptophan zuzusetzen.

Beispiel 3
Das Strukturgen für menschliches β-Interferon wurde aus einer cDNA-Bank gewonnen. Die Klone enthalten die Insertion in die Pst I-Stelle des Plasmids pBR 322. Durch Einwirkung der Restriktionsenzyme Hinf I und Pst I wurde aus dem Strukturgen des β-Interferons ein Abschnitt von 120 bp isoliert. Die Erkennungssequenz der Hinf I-Stelle beginnt dabei 16 Nucleotide nach dem Codon für das N-terminale Methionin des biologisch aktiven β-Interferons.

Das synthetisch gewonnene Oligonucleotid XI

5′ CATGAGCTACAATCTTCTTGG 3′
3′ TCGATGTTAGAAGAACCTAA 5′ (XI)
    NcoI            HinfI

wird mit DNA-Ligase an das Hinf I-Ende des Fragments addiert. Man erhält den DNA-Abschnitt XII.

Aus dem Strukturgen des β-Interferons wurde weiterhin mit Pst I und Bgl II ein DNA-Abschnitt mit 365 bp isoliert. Dieser Abschnitt wurde in das handelsübliche Plasmid pUC 12 kloniert, das vorher mit Pst I und Bam HI umgesetzt wurde. Man erhält das Plasmid pH 188. Nach Amplifikation und Reisolierung wird das Plasmid pH 188 mit Pst I und Eco RI inkubiert und das β-Interferon-Genfragment isoliert (DNA-Abschnitt XIII).

Das Plasmid pH 131/5 wird mit den Restriktionsenzymen Nco I und Eco RI umgesetzt. Anschließend erfolgt die Inkubation der DNA-Abschnitte XII und XIII mit dem geöffneten Plasmid in Anwesenheit des Enzyms DNA-Ligase unter Bedingungen, die zu einer covalenten Verknüpfung der Bindungen führen. Durch Restriktions- und Sequenzanalyse wird die erwartete Sequenz im Plasmid pH 192/5 bestätigt (Figur 3).

Zur Expression des β-Interferons wird analog Beispiel 2 verfahren. Auch hier wird nach Induktion auf dem Elektrophoresegel eine deutliche Bande festgestellt, die bei nicht induzierten Bakterien nicht vorhanden ist. In den Extrakten der Bakterien kann die biologische Aktivität des β-Interferons nachgewiesen werden.

In die Nco I-Stelle des Plasmids pH 131/5 wurde gemäß Beispiel 2 das Strukturgen für Interleukin-2 inseriert. Nach Umsetzung des Plasmids mit Eco RI wurden die überstehenden Enden durch Inkubation mit Klenow-Polymerase in Anwesenheit von Desoxyadenosintriphosphat und Desoxythymidintriphosphat stumpfendig gemacht.

Der handelsübliche Terminator des trp-Operons (Pharmacia P-L Biochemicals) wird in dieses geöffnete, stumpfendige Plasmid unter «blunt end»-Bedingungen und unter gleichzeitigem Ringschluß eingebaut.

Nach Wachstum und Induktion der Bakterien, wie in Beispiel 2 und vorstehend beschrieben, werden die Bakterien abgetrennt und lysiert. SDS-Gelelektrophorese zeigt unverändert die Bande des Interleukin-2-Proteins in der gleichen Intensität wie bei Beispiel 2. Die der β-Lactamase entsprechende Bande weist jedoch eine deutlich geringere Intensität auf.

**Patentansprüche für die Vertragsstaaten: BE, CH, DE, GB, IT, LT, LU, NL, SE**

1. DNA-Abschnitt des trp-Operons aus E. coli, gekennzeichnet durch die Sequenz (codierender Strang) I

5′ GTATCGACC 3′ (I)

die am 5′-Ende an die Shine-Dalgarno-Sequenz AAGG und am 3′-Ende an das Start-Codon ATG anschließt.

2. DNA nach Anspruch 1, gekennzeichnet durch die Sequenz (codierender Strang) Ia

5′ GTATCGACCATGG 3′ (Ia).

die am 5′-Ende an die Shine-Dalgarno-Sequenz AAGG anschließt und bei der das G am 3′-Ende das erste Nucleotid eines Strukturgens nach dem Startcodon ist.

3. Verfahren zur Herstellung eines Vektors mit dem trp-Expressionssystem aus E. coli, dadurch gekennzeichnet, daß man einen E. coli-Vektor, der die DNA-Sequenz I (codierender Strang), gefolgt von 5′ ATGG 3′, enthält, mit dem Restriktionsenzym Nco I schneidet und
a) in die Schnittstelle eine Genstruktur der DNA-Sequenz II

5′ CATGX... 3′
3′        Y... 5′ (II)

in der X und Y das erste komplementäre Nucleotidpaar nach dem Startcodon eines Strukturgens bedeuten, ligiert oder
b) die Schnittstelle enzymatisch auffüllt und die DNA der Sequenz III

5′ GTA TCG ACC ATG 3′
3′ CAT AGC TGG TAC 5′ (III)

mit einer DNA der Sequenz IV

5′ X... 3′ (IV)
3′ Y... 5′

in der X und Y die vorstehende Bedeutung haben, ligiert oder
c) die überstehende Sequenz der Schnittstelle enzymatisch abbaut und die DNA der Sequenz VI

5′ GTA TCG AC 3′ (VI)
3′ CAT AGC TG 5′

mit einer DNA der Sequenz VII

5′ Z ATG X... 3′ (VII)
3′ Z′TAC Y... 5′

ligiert, wobei Z und Z′ ein beliebiges Nucleotidpaar bedeuten, das auch entfallen kann.

4. E. coli, gekennzeichnet durch einen Gehalt an einem gemäß Anspruch 3 erhaltenen Vektor, wobei Z für C und Z′ für G stehen.

5. Verfahren zur Herstellung eines Polypeptids aus genetisch codierbaren Aminosäuren, dadurch gekennzeichnet, daß man E. coli-Zellen gemäß Anspruch 4 zur Expression bringt.

6. Plasmid pH 131/5 gemäß Figur 1.

7. Plasmid pH 185/11 gemäß Figur 2.

**Patentansprüche für den Vertragsstaat Österreich:**

1. Verfahren zur Herstellung eines Vektors mit dem trp-Expressionssystem aus E. coli, dadurch gekennzeichnet, daß man einen E. coli-Vektor, der die DNA-Sequenz I

5′ GTATCGACC 3′       (I)

(codierender Strang), gefolgt von 5′ ATGG 3′, enthält, mit dem Restriktionsenzym Nco I schneidet und
a) in die Schnittstelle eine Genstruktur der DNA-Sequenz II

5′ CATGX... 3′
3′       Y... 5′       (II)

in der X und Y das erste komplementäre Nucleotidpaar nach dem Startcodon eines Strukturgens bedeuten, ligiert oder
b) die Schnittstelle enzymatisch auffüllt und die DNA der Sequenz III

5′ GTA TCG ACC ATG 3′
3′ CAT AGC TGG TAC 5′       (III)

mit einer DNA der Sequenz IV

5′ X... 3′       (IV)
3′ Y... 5′

in der X und Y die vorstehende Bedeutung haben, ligiert oder
c) die überstehende Sequenz der Schnittstelle enzymatisch abbaut und die DNA der Sequenz VI

5′ GTA TCG AC 3′       (VI)
3′ CAT AGC TG 5′

mit einer DNA der Sequenz VII

5′ Z ATG X... 3′       (VII)
3′ Z′TAC Y... 5′

ligiert, wobei Z und Z′ ein beliebiges Nucleotidpaar bedeuten, das auch entfallen kann.
2. Verfahren nach Anspruch 1, dadurch gekennzeichnet, daß die Sequenz (codierender Strang) Ia

5′ GTATCGACCATGG 3′       (Ia),

die am 5′-Ende an die Shine-Dalgarno-Sequenz AAGG anschließt und bei der das G am 3′-Ende das erste Nucleotid eines Strukturgens nach dem Startcodon ist, anstelle der Sequenz I eingesetzt wird.
3. Verfahren zur Herstellung eines Polypeptids aus genetisch codierbaren Aminosäuren, dadurch gekennzeichnet, daß man einen Vektor nach Anspruch 1 oder 2, wobei Z für C und Z′ für G stehen, in E. coli-Zellen zur Expression bringt.

**Claims for the contracting states: BE, CH, DE, GB, IT, LI, LU, NL, SE**

1. DNA segment of the trp operon from E. coli, characterized by the sequence (coding strand) I

5′ GTATCGACC 3′       (I)

which is connected at the 5′ end to the Shine-Dalgarno sequence AAGG, and at the 3′ end to the start codon ATG.
2. DNA according to claim 1, characterized by the sequence (coding strand) Ia

5′ GTATCGACCATGG 3′       (Ia)

which is connected at the 5′ end to the Shine-Dalgarno sequence AAGG, and in which the G at the 3′ end is the first nucleotide of a structural gene downstream of the the start codon.
3. A process for the preparation of a vector having the trp expression system of E. coli, characterized by the cleavage with the restriction enzyme Nco I of an E. coli vector which contains the DNA sequence I (coding strand) followed by 5′ ATGG 3′, and
a) ligation of a gene structure of DNA sequence II

5′ CATGX... 3′       (II)
3′       Y... 5′

in which X and Y denote the first complementary pair of nucleotides downstream of the start codon of a structural gene, in the cleavage site, or
b) enzymatic filling in of the cleavage site and ligation of the DNA of the sequence III

5′ GTA TCG ACC ATG 3′       (III)
3′ CAT AGC TGG TAC 5′

with a DNA of the sequence IV

5′ X... 3′       (IV)
3′ Y... 5′

in which X and Y have the abovementioned meaning, or
c) enzymatic degradation of the protruding sequence of the cleavage site, and ligation of the DNA of the sequence VI

5′ GTA TCG AC 3′       (VI)
3′ CAT AGC TG 5′

with a DNA of the sequence VII

5′ Z′ATG X... 3′       (VII)
3′ Z′TAC Y... 5′

Z and Z′ denoting any desired pair of nucleotides, which can also be dispensed with.
4. E. coli which contains a vector obtained according to in claim 3, Z representing C and Z′ representing G.
5. A process for the preparation of a polypeptide composed of genetically codable amino acids, characterized by bringing about expression of E. coli cells according to claim 4.

6. Plasmid pH 131/5 according to Figure 1.

7. Plasmid pH 185/11 according to Figure 2.

**Claims for the contracting state: AT**

1. A process for the preparation of a vector having the trp expression system of E. coli, characterized by cleavage with the restriction enzyme Nco I of an E. coli vector which contains the DNA sequence I

5′ GTATCGACC 3′     (I)

(coding strand) followed by 5′ ATGG 3′, and
a) ligation of a gene structure of DNA sequence II

5′ CATG X ... 3′     (II)
3′     Y ... 5′

in which X and Y denote the first complementary pair of nucleotides downstream of the start codon of a structural gene, in the cleavage site, or
b) enzymatic filling in of the cleavage site and ligation of the DNA of the sequence III

5′ GTA TCG ACC ATG 3′     (III)
3′ CAT AGC TGG TAC 5′

with a DNA of the sequence IV

5′ X ... 3′     (IV)
3′ Y ... 5′

in which X and Y have the abovementioned meaning, or
c) enzymatic degradation of the protruding sequence of the cleavage site, and ligation of the DNA of the sequence VI

5′ GTA TCG AC 3′     (VI)
3′ CAT AGC TG 5′

with a DNA of the sequence VII

5′ Z ATG X ... 3′     (VII)
3′ Z′ TAC Y ... 5′

Z and Z′ denoting any desired pair of nucleotides, which can also be dispensed with.

2. The process according to claim 1, characterized by sequence (coding strand) Ia

5′ GTATCGACCATGG 3′     (Ia)

which is connected at the 5′ end to the Shine-Dalgarno sequence AAGG, and in which the G at the 3′ end is the first nucleotide of a structural gene downstream of the start codon, is inserted in place of the sequence I.

3. A process for the preparation of a polypeptide composed of genetically codable amino acids, characterized by bringing about expression of a vector according to claim 1 or 2, Z representing C and Z′ representing G, in E. coli cells.

**Revendications pour les Etats contractants: BE, CH, DE, GB, IT, LI, LU, NL, SE**

1. Segment d'ADN de l'opéron trp de E. coli, caractérisé par la séquence (brin codant) I

5′ GTATCGACC 3′     (I)

qui est rattaché par l'extrémité 5′, à la séquence de Shine-Dalgarno AAGG et par l'extrémité 3′ au codon d'initiation ATG.

2. ADN selon la revendication 1, caractérisé par la séquence (brin codant) Ia qui est rattachée, par l'extrémité 5′, à la séquence de Shine-Dalgarno AAGG et dans laquelle le G à l'extrémité 3′ est le premier nucléotide d'un gène de structure après le codon d'initiation.

3. Procédé de production d'un vecteur comportant le système d'expression trp de E. coli, caractérisé en ce que l'on coupe un vecteur de E. coli, qui contient la séquence d'ADN I (brint codant), suivi de 5′ ATGG 3′, avec l'enzyme de restriction Nco I et
a) on lie dans le site de restriction une structure de gène de séquence d'ADN II

5′ GATGX... 3′
3′     Y... 5′     (II)

dans laquelle X et Y représentent la première paire de nucléotides complémentaire après le codon d'initiation d'un gène de structure ou
b) on comble le site de coupure par voie enzymatique et on lie l'ADN de séquence III

5′ GTA TCG ACC ATG 3′
3′ CAT AGC TGG TAC 5′     (III)

avec un ADN de séquence IV

5′ X ... 3′
3′ Y ... 5′     (IV)

dans laquelle X et Y ont la signification ci-dessus ou
c) on dégrade par voie enzymatique la séquence décalée du site de restriction et on lie l'ADN de séquence VI

5′ GTA TCG AC 3′
3′ CAT AGC TG 5′     (VI)

avec un ADN de séquence VII

5′ Z ATG X ... 3′
3′ Z′ TAC Y ... 5′     (VII)

Z et Z' représentant une paire de nucléotides quelconque, qui peut aussi ne pas être présente.

4. E. coli, caractérisé en ce qu'il contient un vecteur obtenu selon la revendication 3, Z représentant C et Z' représentant G.

5. Procédé de production d'un polypeptide consistent d'amino acides codables génétiquement, caractérisé en ce que l'on provoque l'expression de cellules de E. coli selon la revendication 4.

6. Plasmide pH 131/5 selon la figure 1.

7. Plasmide pH 185/11 selon la figure 2.

## Revendications pour l'Etat contractant: AT

1. Procédé de production d'un vecteur comportant le système d'expression trp de E. coli, caractérisé en ce que l'on coupe un vecteur de E. coli, qui contient la séquence d'ADN I (brin codant), suivi de 5' ATGG 3', avec l'enzyme de restriction Nco I et

a) on lie dans le site de restriction une structure de gène de séquence d'ADN II

$$5' \quad GATGX... \quad 3'$$
$$3' \qquad\qquad Y... \quad 5' \qquad (II)$$

dans laquelle X et Y réprésentent la première paire de nucléotides complémentaire après le codon d'initiation d'un gène de structure ou

b) on comble le site de coupure par voie enzymatique et on lie l'ADN de séquence III

$$5' \quad GTA\ TCG\ ACC\ ATG \quad 3'$$
$$3' \quad CAT\ AGC\ TGG\ TAC \quad 5' \qquad (III)$$

avec un ADN de séquence IV

$$5'\ X... \quad 3'$$
$$3'\ Y... \quad 5' \qquad (IV)$$

dans laquelle X et Y ont la signification ci-dessus ou

c) on dégrade par voie enzymatique la séquence décalée du site de restriction et on lie l'ADN de séquence VI

$$5' \quad GTA\ TCG\ AC \quad 3'$$
$$3' \quad CTA\ AGC\ TG \quad 5' \qquad (VI)$$

avec un ADN de séquence VII

$$5'\ Z\ ATG\ X... \quad 3'$$
$$3'\ Z'TAC\ Y... \quad 5' \qquad (VII)$$

Z et Z' représentant une paire de nucléotides quelconque, qui peut aussi ne pas être présente.

2. Procédé selon la revendication 1, caractérisé en ce que la séquence (brin codant) Ia qui se rattache par l'extrémité 5' à la séquence de Shine-Dalgarno AAGG et dans laquelle le G à l'extrémité 3' est le premier nucléotide d'un gène de structure après le codon d'initiation, est employée au lieu de la séquence I.

3. Procédé de production d'un polypeptide consistent d'amino acides codables génétiquement, caractérisé en ce que l'on provoque l'expression dans des cellules de E. coli d'un vecteur selon la revendication 1 ou 2, Z représentant C et Z' représentant G.

Cla I
EcoR1
trp
Hind III
P,0
Ap · ptrp L1 · Tc
pBR 322

Cla I

5' CGACCATGGT    3'
3'    TGGTACCAGC  5'

## FIG. 1

SD                Nco I
5' AAGGGTAT|CG ACCATGGT|CGAT 3'
3' TTCCCATAGC|TGGTACCA GC|TC 5'

Nco I
EcoRI
trp
Hind III
P,0
Ap · pH 131/5 · Tc
pBR 322

SD                β–IF
5' AAGG GTATCGACCATGAGC 3'
3' TTCC CATAGCTGGTACTCG 5'

trp
Hind III
β–IF
pH 192/5
Tc
Ap
EcoRI
pBR 322

## FIG.3

FIG.2

5' AAGGGTATCGAC|CATGGC IL2 CCTGATAG|G 3'
3' TTCCCATAGCTG|GTACCG      GGACTATC|C 5'